# EUROPÄISCHE PATENTSCHRIFT

(11) **EP 2 199 423 B1**
(45) Veröffentlichungstag und Bekanntmachung des Hinweises auf die Patenterteilung: **17.04.2013**
(21) Anmeldenummer: 09173801.3
(22) Anmeldetag: 22.10.2009
(51) Int. Cl.: C23C 4/08, C23C 4/12, C23C 28/00, C23C 30/00, A61L 27/30, A61L 27/32

(54) **Thermische gespritzte Oberflächenschicht, sowie ein orthopädisches Implantat**
Thermally injected surface layer and orthopaedic implant
Couche superficielle extrudée thermique et implant orthopédique

(30) Priorität: 16.12.2008 EP 08171821
(43) Veröffentlichungstag der Anmeldung: 23.06.2010
(73) Patentinhaber: Sulzer Metco AG, 5610 Wohlen (CH)
(72) Erfinder: Zimmermann, Harald, Dr., 5610, Wohlen (CH)
(74) Vertreter: Sulzer Management AG

(56) Entgegenhaltungen:
- EP-A- 1 449 930
- EP-A- 1 806 154
- WO-A-99/58167
- US-A1- 2008 008 654
- US-B1- 6 953 560

## Beschreibung

Die Erfindung betrifft eine thermisch gespritzte Oberflächenschicht aus Titan auf einem nicht-metallischen Substrat eines orthopädischen Implantats, sowie ein orthopädisches Implantat gemäss dem Oberbegriff des jeweiligen unabhängigen Anspruchs.

Orthopädische Implantate werden heute häufig aus Titanlegierungen, Edelstahl oder CoCrMo-Legierungen hergestellt. Zum bessren Anwachsen des Knochens und zur Beschleunigung des Heilungsprozesses werden die Implantate in der Regel mit einer porösen Titanschicht und / oder einer biokompatiblen Hydroxylapatitschicht versehen. Die vorgenannten Metalle und Metalllegierungen lassen sich zum Beispiel in einer Röntgenaufnahme hervorragend lokalisieren, so dass der Chirurg den Erfolg seiner Arbeit unmittelbar anhand einer Röntgenaufnahme beurteilen kann.

Beispielweise in der EP A 1 449 930 oder der US 2008/008 654 A1 sind bereits orthopädische Implantate mit einer Oberflächenschicht enthaltend Titan mit Scandium und mehreren röntgensensitiven Indikatormetallen beschrieben.

Als neuer Substratwerkstoff für orthopädische Implantate haben in letzter Zeit mehr und mehr nicht-metallische Werkstoffe an Bedeutung gewonnen. Ein sehr wichtiger Werkstoff, der sehr gut biokompatibel ist und daher sehr breit in allen möglichen medizinischen Anwendungen erfolgreich eingesetzt wird, ist das bestens bekannte Polyetheretherketon (PEEK), wobei insbesondere für orthopädische Anwendungen ein entsprechendes Substrat aus PEEK mit Kohlefasern verstärkt werden kann. Für spezielle Anwendungen kommen natürlich auch andere Kunststoffwerkstoffe in Frage. Auch sind im Prinzip Substrate möglich, die ganz aus einem Kohlefaserwerkstoff aufgebaut sind, oder aus anderen nicht-metallischen Werkstoffen bestehen.

Der Nachteil dieser an sich sehr innovativen Werkstoffe besteht darin, dass sie in einer Röntgenaufnahme nicht zu erkennen sind, weil sie praktisch keine schweren Metallatome enthalten. Selbst dann, wenn die Substrate mit einer Titanbeschichtung, zum Beispiel mittels einer dünnen thermischen Titanbeschichtung oder mit einer Hydroxylapatitschicht versehen sind, bleiben die Implantate im Röntgenbild weitgehend unsichtbar. Das verhältnismässig leichte Titanatom hat nämlich für Röntgenlicht einen verhältnismässig kleinen Wechselwirkungsquerschnitt, so dass es in einer dünn gespritzten thermischen Spritzschicht auf einer Röntgenaufnahme praktisch nicht zu sehen ist.

Damit der Chirurg dennoch ein eingepflanztes Implantat auf einer Röntgenaufnahme lokalisieren kann, ist es bekannt, die meist spritzgegossenen Implantatrohlinge bzw. Substrate mit den ebenfalls biokompatiblen Tantaldrähten zu versehen. Da Tantal ein rund viermal höheres Atomgewicht als Titan hat, ist Tantal deutlich besser, auch in relativ kleinen Mengen, auf einer Röntgenaufnahme zu sehen.

Es liegt auf der Hand, dass die Präparation der Implantate mit Tantaldrähten eine sehr aufwändige, komplizierte und damit kostspielige Angelegenheit ist. Ausserdem sind die so präparierten Implantate auf den Röntgenaufnahmen auch nur grob lokalisierbar, weil die Ortsauflösung letztendlich durch den gegenseitigen Abstand benachbarter Tantaldrähte beschränkt ist. Um die Ortsauflösung zu erhöhen, muss die Dichte der Tantaldrähte im Substrat erhöht werden, was den Aufwand nur noch höher treibt.

Im Prinzip könnten die Implantate auch mit einer thermischen Tantalbeschichtung durch thermisches Spritzen beschichtet werden. Aber das verbietet sich, weil nicht nur die Biokompatibilität des Materials als solches eine Rolle spielt. Auch die Oberflächenstruktur hat entscheidenden Einfluss auf das Anwachsen des Knochens an die Oberfläche des Implantats und auf den Heilungsprozess. Daher ist möglichst eine mehr oder weniger reine Tantaloberfläche zu vermeiden.

Vielmehr ist eine Titanoberfläche gewünscht, die nicht nur aus chemischer Sicht eine hervorragende Biokompatibilität hat, sondern eine spezielle Oberflächenstruktur aufweist, die das Anwachsen an den Knochen und den Heilungsprozess enorm befördern. Somit gibt es bisher bei orthopädischen Implantaten, die ein nicht-metallisches Substrat haben, für die Verwendung der Tantaldrähte keine vernünftige Alternative zur Lokalisierung des Implantats im menschlichen oder tierischen Körper.

Es ist daher die Aufgabe der Erfindung, eine thermisch gespritzte Oberflächenschicht aus Titan auf einem nicht-metallischen Substrat bereit zustellen, die alle bekannten hervorragenden biokompatiblen Eigenschaften der einer konventionellen Titanbeschichtung hat und gleichzeitig zur Lokalisierung des Substrats im menschlichen oder tierischen Körper auf einer Röntgenaufnahme ausreichend gut mit hoher Ortsauflösung zu erkennen ist.

Eine weitere Aufgabe der Erfindung ist es, ein entsprechendes orthopädisches Implantat mit einer thermisch gespritzten Oberflächenschicht aus Titan bereitzustellen.

Die diese Aufgaben lösenden Gegenstände der Erfindung sind durch die Merkmale des jeweiligen unabhängigen Anspruchs gekennzeichnet.

Die zugeordneten abhängigen Ansprüche beziehen sich auf besonders vorteilhafte Ausführungsformen der Erfindung.

Die Erfindung betrifft somit eine thermisch gespritzte Oberflächenschicht aus Titan auf einem nicht-metallischen Substrat eines orthopädischen Implantats, wobei die thermisch gespritzte Oberflächenschicht in Bezug auf Titan eine röntgensensitive Beimischung zwischen 0.01 at% und 20 at% aus einem biokompatiblen Indikatormetall umfasst, und das Indikatormetall ein Metall aus der Gruppe der Metalle bestehend aus Tantal, Gold, Platin und Hafnium ist, so dass ein Atomgewicht des biokompatiblen Indikatormetalls grösser ist als das Atomgewicht von Titan. Erfindungsgemäss ist die thermisch gespritzte Oberflächenschicht als Titanmatrix mit Bereichen aus eingelagertem Indikatormetall ausgebildet.

Die vorliegende Erfindung nutzt also gleichzeitig die hervorragenden biokompatiblen Eigenschaften der Titanbeschichtung und die röntgensensitiven Eigenschaften eines biokompatiblen Indikatormetalls, das in einer relativ kleinen Konzentration als Beimischung in der Oberflächenschicht aus Titan vorgesehen ist, die mittels eines thermischen Spritzprozesses auf das nicht-metallische Substrat thermisch aufgespritzt ist. Dadurch, dass das röntgensensitive Indikatormetall nur als Beimischung in relativ kleiner Konzentration in der Titanbeschichtung vorgesehen ist, bleibt die eigentliche Struktur der Titanbeschichtung im wesentlichen unverändert erhalten, so dass die so aufgebaute Schicht nicht nur hervorragend in einer Röntgenaufnahme anhand des Indikatormetalls lokalisierbar ist. Gleichzeitig hat die erfindungsgemässe Oberflächenschicht alle hervorragenden biokompatiblen Eigenschaften der bekannten konventionellen Titan Oberflächenschichten, so dass insbesondere sowohl ein optimales Anwachsen des Knochen an die erfindungsgemässe Oberflächenschicht, als auch die gewünschte Beschleunigung des Heilungsprozesses durch die Titanstruktur uneingeschränkt gewährleistet sind.

Das Titanpulver, das beim thermischen Beschichten verwendet wird, kann dazu einfach mit einer relativ kleinen Konzentration eines Indikatormetalls sozusagen verunreinigt werden. Ein wichtiges Beispiel eines Indikatormetalls ist dabei Tantal, das ein in etwa vierfach höheres Atomgewicht als Titan hat und daher auch in kleinen Konzentrationen hervorragend in einer Röntgenaufnahme lokalisierbar ist.

Ein weiterer wesentlicher Vorteil ist, dass keine Legierungsbildung zwischen dem Titan und dem Indikatormetall notwendig ist. Vielmehr werden bevorzugt einfach das Titanpulver und ein Pulver aus Indikatormetall gleichzeitig thermisch gespritzt, wobei einfach kommerziell erhältliche Spritzpulver verwendet werden können, die vor dem thermischen Spritzen nicht weiter aufbereitet werden müssen. Insbesondere ist es nicht notwendig, die beiden Pulver miteinander zu legieren oder einer speziellen Mischungsprozedur zu unterwerfen. Die Pulver können, sofern sie ansonsten die medizinisch gebotenen Spezifikationen erfüllen, ohne weitere Bearbeitung thermisch auf das nicht-metallische Substrat des Implantats aufgespritzt werden.

Somit kann durch die vorliegende Erfindung bei der Verwendung von nicht-metallischen Substraten erstmals auf die aufwändige Technik der Präparation der Implantate mit Tantaldrähten verzichtet werden.

Damit die strukturellen Eigenschaften der Titanbeschichtung durch eine zu grosse Beimischung von Tantal nicht gestört wird, gleichzeitig jedoch das Indikatormetall in der Röntgenaufnahme noch in ausreichender Deutlichkeit und Ortsauflösung sichtbar ist, hat sich gezeigt, dass ein Gehalt an Indikatormetall in Bezug auf die Gesamtmenge an Titan in der Oberflächenschicht zwischen 0.01 Atomprozent (at%) und 20 Atomprozent (at%) Tantal enthalten sein sollte. Ist deutlich weniger Tantal als 0.01 at% in der Titanschicht enthalten, wird die Lokalisierbarkeit des Implantats auf einer Röntgenaufnahme unbrauchbar schlecht. Werden deutlich mehr als 20 at% der Titanoberfläche beigemischt, verändert sich die Struktur der Titanschicht zunehmend derart, das das Anwachsen des Knochens an das Implantat nicht mehr ausreichend befördert wird und auch der Heilungsprozess nicht mehr wie gewünscht unterstützt wird.

Bevorzugt liegt der Anteil des Indikatormetalls sogar nur zwischen 0.1 at% und 10 at%, je nach verwendetem Indikatormetall zwischen 0.5 at% und 5 at%.

Das Indikatormetall ist dabei ein Metall aus der Gruppe der Metalle bestehend aus Tantal, Gold, Platin und Hafnium ist. Alle vorgenannten Metalle haben die geforderten biokompatiblen Eigenschaften und gleichzeitig ein ausreichend grosses Atomgewicht, so dass sie sich als Indikatormetall im Sinne der vorliegenden Erfindung hervorragend eignen.

Auch wenn eine Legierung des Indikatormetalls mit dem Titan der Oberflächenschicht nicht notwendig ist, kann das Indikatormetall natürlich trotzdem ganz oder teilweise mit dem Titan legiert sein.

Dabei kann das Indikatormetall selbst eine Legierung aus zwei oder mehr Metallen aus der Gruppe der Metalle bestehend aus Tantal, Gold, Platin und Hafnium sein.

In einem für die Praxis besonders wichtigen Ausführungsbeispiel besteht die die thermisch gespritzte Oberflächenschicht bis auf medizinisch und technisch unbedeutende Verunreinigungen nur aus Titan und dem Indikatormetall. Dadurch ist gewährleistet, dass die erfindungsgemässe Oberflächenschicht keine medizinisch kritischen Fremdstoffe enthält.

In einem weiteren wichtigen Ausführungsbeispiel ist die thermisch gespritzte Oberflächenschicht als Titanmatrix mit Bereichen aus eingelagertem Indikatormetall ausgebildet. Das heisst, die Struktur der Oberflächenschicht ist derart, dass in eine Schicht aus Titan kleine Bereiche, bevorzugt lokal isolierte Bereiche bestehend aus dem Indikatormetall eingebettet sind. Das kann zum Beispiel dadurch erreicht werden, wenn der thermische Spritzprozess so geführt wird, dass das Indikatormetall beim thermischen Spritzprozess im wesentlichen nicht aufgeschmolzen, höchstens vielleicht an der Oberfläche der Pulverkörner angeschmolzen wird und daher als isolierte Bereiche in der Titanmatrix eingebaut werden.

Die Erfindung betrifft weiter ein orthopädisches Implantat umfassend ein nicht-metallisches Substrat mit einer thermisch gespritzten Oberflächenschicht aus Titan, wobei die thermisch gespritzte Oberflächenschicht in Bezug auf Titan eine röntgensensitive Beimischung zwischen 0.01 at% und 20 at% aus einem biokompatiblen Indikatormetall umfasst, und das Indikatormetall ein Metall aus der Gruppe der Metalle bestehend aus Tantal, Gold, Platin und Hafnium ist, so dass ein Atomgewicht des biokompatiblen Indikatormetalls grösser ist als das Atomgewicht von Titan. Erfindungsgemäss ist die thermisch gespritzte Oberflächenschicht als Titanmatrix mit Bereichen aus eingelagertem Indikatormetall ausgebildet.

Bevorzugt liegt der Anteil des Indikatormetalls sogar nur zwischen 0.1 at% und 10 at%, je nach verwendetem Indikatormetall zwischen 0.5 at% und 5 at%, wobei das Indikatormetall ein Metall aus der Gruppe der Metalle bestehend aus Tantal, Gold, Platin und Hafnium ist, wobei das Indikatormetall auch eine Legierung aus zwei oder mehr Metallen aus der Gruppe der Metalle bestehend aus Tantal, Gold, Platin und Hafnium sein kann.

Besonders bevorzugt besteht die thermisch gespritzte Oberflächenschicht bis auf Verunreinigungen nur aus Titan und dem Indikatormetall, wobei die thermisch gespritzte Oberflächenschicht besonders vorteilhaft, aber nicht notwendig als Titanmatrix mit Bereichen aus eingelagertem Indikatormetall ausgebildet ist.

Das orthopädische Implantat der vorliegenden Erfindung umfasst dabei bevorzugt ein Substrat aus einem biokompatiblen Kunststoff, insbesondere aus PEEK und / oder aus einem Kohlefaserwerkstoff, insbesondere aus einem PEEK-Kohlefaser Verbundwerkstoff.

Vorteilhaft kann auf der thermisch gespritzten Oberflächenschicht eine biokompatible Hydroxylapatitschicht vorgesehen sein, die zusätzlich zum porösen Titan zur Beschleunigung des Heilungsprozesses beiträgt und das Anwachsen an den Knochen ebenfalls positiv unterstützt.

Im speziellen kann ein orthopädisches Implantat der vorliegenden Erfindung zum Beispiel ein Schulter-Implantat, ein Ellbogen-Implantat, ein Wirbelsäulen-Implantat, eine Hüftgelenk-Implantat, ein Knie-Implantat, ein Finger-Implantat, oder ein anderes orthopädisches Implantat sein.

Im Folgenden wird die Erfindung an Hand der schematischen Zeichnung näher erläutert. Es zeigen:
- Fig. 1: eine Hüftgelenkpfanne mit einer erfindungsgemässen Oberflächenschicht;
- Fig. 2: die Oberflächenschicht gemäss Fig. 1 im Schnitt;
- Fig. 2a: ein Implantat mit Oberflächenschicht und Haftschicht;
- Fig. 2b: ein Implantat gemäss Fig. 2 mit einer Indikatorschicht;
- Fig. 3: ein bekanntes thermisches Spritzverfahren mit verschiedenen Spritzwerkstoffen;
- Fig. 4: ein Spritzverfahren mit verschiedenen Spritzwerkstoffen zur Herstellung einer erfindungsgemässen Oberflächenschicht.

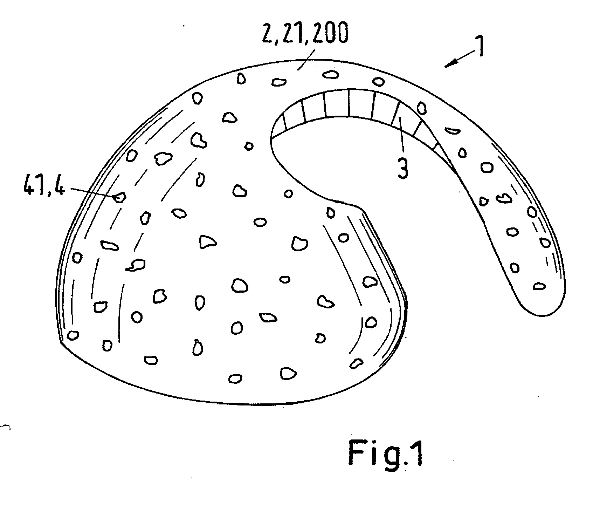

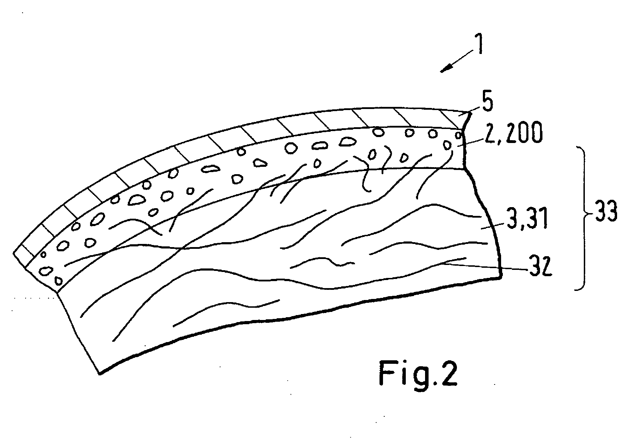

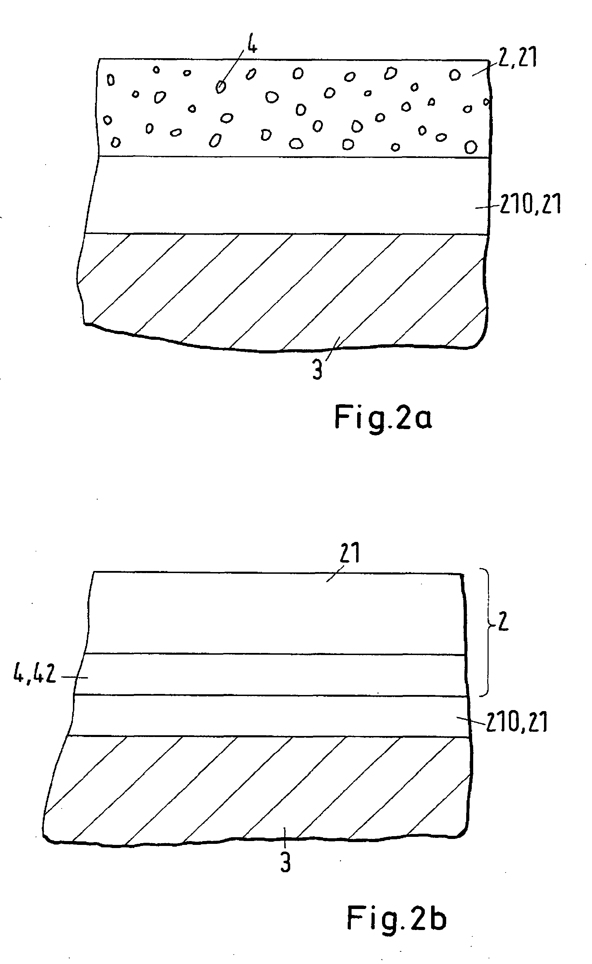

In Fig. 1 ist in einer perspektivischen Darstellung eine Hüftgelenkpfanne mit einer erfindungsgemässen Oberflächenschicht schematisch dargestellt. Fig. 2 zeigt die Oberflächenschicht gemäss Fig. 1 im Schnitt.

Die Hüftgelenkpfanne 1 gemäss Fig. 1 bzw. Fig. 2 umfasst ein nicht-metallisches Substrat 3 mit einer thermisch gespritzten Oberflächenschicht 2 aus Titan 21. Die thermisch gespritzte Oberflächenschicht 2 der Fig. 1 und 2 weist in Bezug auf Titan 21 eine röntgensensitive Beimischung von ca. 2 at% bzw. 5 at% aus einem biokompatiblen Indikatormetall 4, wobei im vorliegenden Beispiel das Indikatormetall 4 Tantal 4 ist, dessen Atomgewicht ca. viermal grösser ist als das Atomgewicht von Titan, so dass die Hüftgelenkpfanne 1 in einer Röntgenaufnahme sehr gut vom Chirurgen lokalisierbar ist.

Die thermisch gespritzte Oberflächenschicht 2 besteht dabei bis auf technisch und medizinisch nicht relevante Verunreinigungen nur aus Titan 21 und Tantal 4.

Wie gut zu erkennen ist, ist die thermisch gespritzte Oberflächenschicht 2 als Titanmatrix 200 mit Bereichen 41 aus eingelagertem Tantal 4 ausgebildet.

Das Substrat 3 ist im Beispiel der Fig. 1 bzw. Fig. 2 aus einem PEEK-Kohlefaser Verbundwerkstoff 33 aufgebaut. D.h., das Substrat 3 der Hüftgelenkpfanne 1 besteht aus PEEK 31, in welches in an sich bekannter Weise zur Verstärkung Kohlefasern 32 eingebettet sind.

Wie gut zu erkennen ist, ragen die Kohlefasern 32 aus der Oberfläche des Substrats 3 teilweise heraus. Die thermische Oberflächenschicht 2 dient somit gleichzeitig dazu, die herausstehenden Kohlefasern 32 zu binden, so dass die herausstehenden 32 nicht in schädliche Wechselwirkung mit dem Geweben eines menschlichen oder tierischen Körpers treten können, in den die Hüftgelenkpfanne 1 implantiert wird.

Zusätzlich ist auf der thermisch gespritzten Oberflächenschicht 2 eine biokompatible Hydroxylapatitschicht 5 vorgesehen, die zusätzlich das Anwachsen des Knochens an die Hüftgelenkpfanne 1 und die Heilung allgemein positiv beeinflusst.

Die Fig. 2a und Fig. 2b zeigen beispielhaft ein Implantat 1 mit weiteren Varianten von thermisch gespritzten Oberflächenschichten 2 gemäss der vorliegenden Erfindung.

In Fig. 2a ist schematisch im Schnitt ein orthopädisches Implantat 1 gezeigt, bei welchem zwischen der erfindungsgemässen Oberflächenschicht 2 und dem Substrat 3 noch eine Haftschicht 210 vorgesehen ist, die im vorliegenden Beispiel aus Titan 21 besteht. Durch das Vorsehen der Haftschicht 210, die besser auf dem Substrat 3 haftet als die erfindungsgemässe Oberflächenschicht 2, und gleichzeitig hervorragend an der Oberflächenschicht 2 haftet, wird die Haftung der Oberflächenschicht 2 auf dem Substrat 3 des Implantats 1 deutlich verbessert.

In Fig. 2b ist ein weiteres spezielles Ausführungsbeispiel einer erfindungsgemässen Oberflächenschicht 2 schematisch dargestellt, die hier durch ein Zweischichtsystem aus einer äusseren Oberflächenschicht aus reinem Titan 21 und einer darunterliegenden Indikatorschicht 42, die im wesentlichen nur aus dem Indikatormetall 4 besteht, gebildet ist. Zwischen der Indikatorschicht 42 und dem Substrat 3 ist wieder eine Haftschicht 210 aus Titan 21 vorgesehen, weil die Haftschicht 210 aus Titan 21 besser auf dem Substrat 3 haftet als die Indikatorschicht 42 und gleichzeitig sehr gut an der Indikatorschicht 42 haftet. Hier ist also die Beimischung aus dem biokompatiblen Indikatormetall dadurch realisiert, dass ein Zweischichtsystem aus der Indikatorschicht 42 und der äusseren Schicht aus Titan 21 gebildet ist.

Dabei kann eine Haftschicht 210 auch dann vorgesehen werden, wenn zum Beispiel der Schmelzpunkt des Indikatormetalls 4 vergleichsweise hoch liegt. Dann ist es möglich, dass zum Beschichten des Substrats 3 mit dem Indikatormetall 4 eine Flammenergie im thermischen Spritzprozess entsprechend so hoch gewählt werden müsste, dass das Substrat 3, das zum Beispiel aus einem temperaturempfindlichen Kunststoff besteht, beim Spritzprozess beschädigt würde. In dem Fall wird vorteilhaft zunächst auf das Substrat 3 eine Haftschicht 210 aus einem Material aufgebracht, das einen niedrigeren Schmelzpunkt als das Indikatormetall 4 hat. Dadurch ist die Oberfläche des Substrats 3 durch die Haftschicht 210 geschützt, auf die dann zum Beispiel eine Indikatorschicht 42 aus dem höher schmelzenden Indikatormetall 4 aufgebracht wird.

Der Fachmann versteht, dass selbstverständlich auch andere Kombinationen von Schichten zu Mehrschichtsystemen vorteilhaft gebildet werden können und dass z.B. für die Haftschicht auch andere Werkstoffe als Titan vorteilhaft einsetzbar sind.

Zur Herstellung einer erfindungsgemässen Oberflächenschicht 2 muss dabei jedoch ein besonderes thermisches Spritzverfahren angewendet werden. Der Grund dafür liegt darin, dass als Spritzmaterial einfach zwei verschiedene kommerziell erhältliche Pulver gemischt werden.

Je nach den speziellen Anforderungen an das Substrat ist es möglich, dass die Massen der einzelnen Pulverteilchen der beiden Spritzpulver deutlich verschieden sind. Das wird in der Regel dann der Fall sein, wenn die Pulverteilchen des Titanspritzpulvers und des Spritzpulvers des Indikatormetalls, also zum Beispiel Tantal, ungefähr die gleiche Grösse bzw. die gleiche Grössenverteilung haben.

Für viele Anwendungen ist das zusammen mit einer gleichen Morphologie der beiden Spritzpulver von besonderem Vorteil, weil sonst leicht eine Entmischung entstehen kann, so dass die Bereiche aus dem Indikatormetall sehr ungleichmässig in der metallischen Matrix aus Titan verteilt sind. Das gilt es natürlich zu verhindern. Das heisst, sind zum Beispiel die Pulverkörner beider Pulver kugelförmig oder beide sind eckig oder haben eine kantige gebrochene Struktur. Wenn zu dem beide Pulversorten zusätzlich Pulverkörner mit vergleichbarer Grösse haben, ist eine Entmischung der beiden Pulver während des thermischen Spritzens eher nicht zu erwarten.

Da aber das Indikatormetall ein höheres Atomgewicht als Titan haben muss, haben zwangsläufig die Pulverkörner des Indikatormetalls eine grössere Masse als die Titankörner, wenn die beiden verschiedenen Pulverkörner ungefähr gleich Grösse haben.

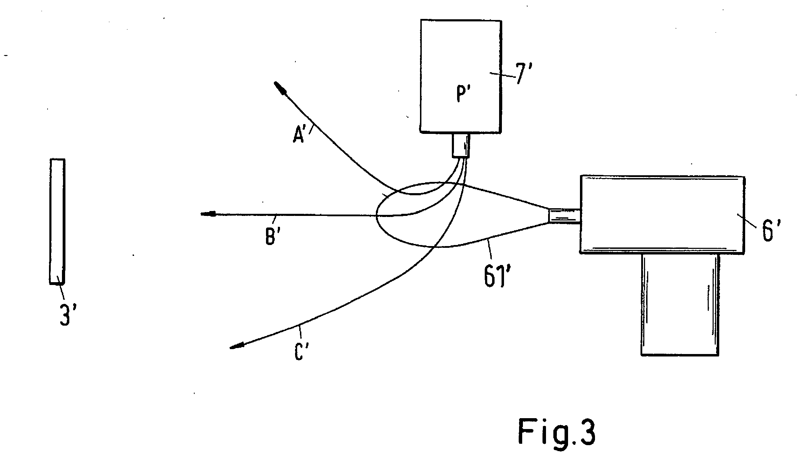

Das kann bei Spritzen zu ernsten Komplikationen führen, wie eindrücklich anhand der Fig. 3 demonstriert wird. Die Fig. 3 zeigt einen aus dem Stand der Technik bekannten Spritzprozess, der mit drei verschiedenen Sorten A', B', und C' von Pulverkörnern durchgeführt wird, die zwar ungefähr die gleich Grösse aber deutlich verschiedene Massen haben.

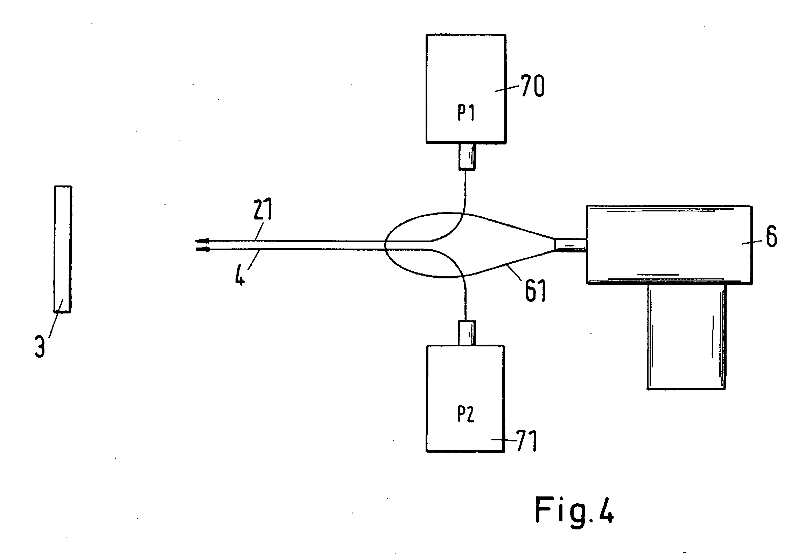

Zur deutlichen Unterscheidung der Erfindung vom Stand der Technik sind in Fig. 3 die aus dem Stand der Technik bekannten Merkmale mit einem Hochkomma versehen, während die Merkmale der vorliegenden Erfindung kein Hochkomma tragen. Damit auch nicht die Merkmale gemäss Fig. 4, die ein Verfahren zur Erzeugung einer erfindungsgemässen thermischen Oberflächenschicht aus Titan zeigt.

Bei dem in Fig. 3 dargestellten Spritzvorgang werden drei verschiedene Spritzpulver A', B', und C' durch den Pulverinjektor 7' der Plasmaflamme 61' der Spritzpistole 6' gleichzeitig zugeführt. Der Pulverinjektor 7' wird dabei mit einem Arbeitsdruck P' betrieben unter dem die Pulverteilchen A', B', und C' in die Plasmaflamme 61' injiziert werden.

Dabei sind die Pulverkörner A' leichter als die Pulverkörner B' und diese wiederum leichter als die Pulverkörner C'.

Das führt dazu, dass der Arbeitsdruck P', unter dem die Pulverteilchen A', B', C' in die Plasmaflamme 61' injiziert werden, nur für die Teilchenmasse einer Teilchensorte, im vorliegenden Fall für die Masse der Teilchen B' angepasst und somit optimal sein kann.

Für die leichteren Teilchen A' ist der Arbeitsdruck P' zu klein. Diese werden quasi an der Plasmaflamme 61' reflektiert und können zumindest nur in unzureichender Menge in die Plasmaflamme 61' eingebracht werden. Die Masse der Teilchen B' wiederum ist für den Arbeitsdruck P' zu gross, so dass die Teilchen B' zumindest teilweise durch die Plasmaflamme 61' hindurch geschleudert werden.

Das negative Ergebnis ist klar: In der thermischen Spritzschicht, die auf das Substrat 3' aufgespritzt werden soll, ist anteilsmässig zu viel Material der Sorte B' vorhanden, während zu wenig Material der Sorte A' und C' in der thermischen Spritzschicht vorhanden sein wird.

Die vorliegende Erfindung vermeidet dieses Problem, indem gemäss Fig. 4 zur Herstellung einer erfindungsgemässen Oberflächenschicht 2 zum Beispiel zwei Pulverinjektoren 70 und 71 verwendet werden. Der Pulverinjektor 70 stellt dabei Titanpulver 21 zur Verfügung und der Pulverinjektor 71 das Indikatormetall 4, wie durch die entsprechenden Pfeile in Fig. 4 angedeutet. Dabei können natürlich auch zwei Pulverinjektoren 70, 71 in einem einzigen Injektormodul untergebracht sein.

Die Arbeitsdrücke mit denen die beiden Pulverinjektoren 70 und 71 betrieben werden sind dabei verschieden und auf die unterschiedlichen Massen von Titanpulver 21 und Indikatormetallpulver 4 optimal abgestimmt, so dass beide Pulversorten 21, 4 optimal in die Plasmaflamme 61 der Plasmaspritzpistole 6 injiziert werden, so dass auf dem Substrat 3 eine erfindungsgemässe thermisch gespritzte Oberflächenschicht in hoher Qualität und der gewünschten Zusammensetzung herstellbar ist.

In einer anderen Variante wird eine Pulvermischung aus dem Titan und dem Indikatormetall hergestellt, wobei die Korngrössenverteilung und die Morphologie von Titan und / oder Indikatormetall so angepasst werden, dass das Pulver über einen einzigen Pulverinjektor mit einem einheitlichen Arbeitsdruck der Plasmaflamme zuführbar ist. Das geschieht vorzugsweise dadurch, dass man zum Beispiel grössere Titan Pulverkörner mit kleineren Indikatorpulverkörnern mischt, wobei die Grösse bzw. die Grössenverteilung der beiden Teilchensorten so gewählt wird, dass die Masse bzw. die Massenverteilung beider Teilchensorten so aufeinander abgestimmt sind, dass beide mittels des gleichen Pulverinjektors und unter dem gleichen Arbeitsdruck optimal in die Plasmaflamme einbringbar sind. In einem bevorzugten Ausführungsbeispiel wird die Grösse bzw. die Grössenverteilung der beiden Teilchensorten so gewählt, dass die Masse bzw. die Massenverteilung beider Teilchensorten ungefähr gleich ist.

Wie bereits erwähnt, müssen in einem bevorzugten Ausführungsbeispiel dabei die kleinen Indikatormetallkörner, zum Beispiel die kleinen Tantalkörner beim thermischen Spritzprozess nicht unbedingt aufschmelzen, sondern sie können in die sich bildende Titanmatrix eingeschlossen werden. Bevorzugt ist dabei an einem Pulverförderer, der das Spritzpulver zum Pulverinjektor fördert, ein Rührwerk vorgesehen, so dass das Spritzpulver ständig gut durchmischt ist, bevor die Pulvermischung aus Titan und Indikatormetall in die Plasmaflamme injiziert wird.

## Patentansprüche

1. Thermisch gespritzte Oberflächenschicht aus Titan (21) auf einem nicht-metallischen Substrat (3) eines orthopädisches Implantats (1), wobei die thermisch gespritzte Oberflächenschicht in Bezug auf Titan (21) eine röntgensensitive Beimischung zwischen 0.01 at% und 20 at% aus einem biokompatiblen Indikatormetall (4) umfasst, und wobei das Indikatormetall (4) ein Metall aus der Gruppe der Metalle bestehend aus Tantal, Gold, Platin und Hafnium ist, so dass ein Atomgewicht des biokompatiblen Indikatormetalls (4) grösser ist als das Atomgewicht von Titan (21), **dadurch gekennzeichnet, dass** die thermisch gespritzte Oberflächenschicht als Titanmatrix (200) mit Bereichen (41) aus eingelagertem Indikatormetall (4) ausgebildet ist.

2. Thermisch gespritzte Oberflächenschicht nach Anspruch 1, wobei der Anteil des Indikatormetalls (4) zwischen 0.5 at% und 5 at% liegt.

3. Thermisch gespritzte Oberflächenschicht nach Anspruch 2, wobei das Indikatormetall (4) eine Legierung aus zwei Metallen aus der Gruppe der Metalle bestehend aus Tantal, Gold, Platin und Hafnium ist.

4. Thermisch gespritzte Oberflächenschicht nach einem der vorangehenden Ansprüche, wobei die thermisch gespritzte Oberflächenschicht bis auf Verunreinigungen aus Titan (21) und dem Indikatormetall (4) besteht.

5. Orthopädisches Implantat umfassend ein nicht-metallisches Substrat (3) mit einer thermisch gespritzten Oberflächenschicht (2) aus Titan (21), wobei die thermisch gespritzte Oberflächenschicht (2) in Bezug auf Titan (21) eine röntgensensitive Beimischung zwischen 0.01 at% und 20 at% aus einem biokompatiblen Indikatormetall (4) umfasst, wobei das Indikatormetall (4) ein Metall aus der Gruppe der Metalle bestehend aus Tantal, Gold, Platin und Hafnium ist, so dass ein Atomgewicht des biokompatiblen Indikatormetalls (4) grösser ist als das Atomgewicht von Titan (21), **dadurch gekennzeichnet, dass** die thermisch gespritzte Oberflächenschicht als Titanmatrix (200) mit Bereichen (41) aus eingelagertem Indikatormetall (4) ausgebildet ist.

6. Orthopädisches Implantat nach Anspruch 5, wobei der Anteil des Indikatormetalls (4) zwischen 0.5 at% und 5 at% liegt.

7. Orthopädisches Implantat nach Anspruch 6, wobei das Indikatormetall (4) eine Legierung aus zwei Metallen aus der Gruppe der Metalle bestehend aus Tantal, Gold, Platin, und Hafnium ist.

8. Orthopädisches Implantat nach einem der Ansprüche 5 bis 7, wobei die thermisch gespritzte Oberflächenschicht (2) bis auf Verunreinigungen aus Titan (21) und dem Indikatormetall (4) besteht.

9. Orthopädisches Implantat nach einem der Ansprüche 5 bis 8, wobei das Substrat (3) einen biokompatiblen Kunststoff, insbesondere PEEK (31) und / oder einen Kohlefaserwerkstoff (32), insbesondere einen PEEK-Kohlefaser Verbundwerkstoff (33) umfasst.

10. Orthopädisches Implantat nach einem der Ansprüche 5 bis 9, wobei auf der thermisch gespritzten Oberflächenschicht (2) eine biokompatible Hydroxylapatitschicht (5) vorgesehen ist.

11. Orthopädisches Implantat nach einem der Ansprüche 5 bis 10, wobei das orthopädisches Implantat ein Schulter-Implantat, ein Ellbogen-Implantat, ein Wirbelsäulen-Implantat, eine Hüftgelenk-Implantat, ein Knie-Implantat, ein Finger-Implantat, oder ein anderes orthopädisches Implantat ist.

## Claims

1. A thermally sprayed surface layer of titanium (21) on a non-metal substrate (3) of an orthopedic implant (1), wherein the thermally sprayed surface layer includes an X-ray sensitive admixture with respect to the titanium (21) which X-ray sensitive admixture includes a biocompatible indicator metal (4) between 0.01 at% and 20 at%, and wherein the indicator metal (4) is a metal from the group of metals including tantalum, gold, platinum and hafnium, so that an atomic weight of the biocompatible indicator metal (4) is larger than the atomic weight of titanium (21), **characterized in that** the thermally sprayed surface layer is formed as a titanium matrix (200) having regions (41) of embedded indicator metal (4).

2. A thermally sprayed surface layer in accordance with claim 1,
wherein the proportion of the indicator metal (4) lies between 0.5 at% and 5 at%.

3. A thermally sprayed surface layer in accordance with claim 2,
wherein the indicator metal (4) is an alloy of two metals from the group of metals including tantalum, gold, platinum and hafnium.

4. A thermally sprayed surface layer in accordance with any one of the preceding claims, wherein the thermally sprayed surface layer includes, apart from any contaminants, titanium (21) and the indicator metal (4).

5. An orthopedic implant including a non-metal substrate (3) with a thermally sprayed surface layer (2) of titanium (21), wherein the thermally sprayed surface layer (2) includes an x-ray sensitive admixture with respect to the titanium (21) which x-ray sensitive admixture includes a biocompatible indicator metal (4) between 0.01 at% and 20 at%, wherein the indicator metal (4) is a metal from the group of metals including tantalum, gold, platinum and hafnium, so that an atomic weight of the biocompatible indicator metal (4) is larger than the atomic weight of titanium (21), **characterized in that** the thermally sprayed surface layer is formed as a titanium matrix (200) having regions (41) of embedded indicator metal (4).

6. An orthopedic implant in accordance with claim 5, wherein the proportion of the indicator metal (4) lies between 0.5 at% and 5 at%.

7. An orthopedic implant in accordance with claim 6, wherein the indicator metal (4) is an alloy of two metals from the group of metals including tantalum, gold, platinum and hafnium.

8. An orthopedic implant in accordance with any one of claims 5 to 7, wherein the thermally sprayed layer (2) includes, apart from any contaminants, titanium (21) and the indicator metal (4).

9. An orthopedic implant in accordance with any one of claims 5 to 8, wherein the substrate (3) includes a biocompatible plastic, in particular PEEK (31) and / or a carbon fiber material (32), in particular a PEEK carbon fiber composite material (33).

10. An orthopedic implant in accordance with any one of claims 5 to 9, wherein a biocompatible hydroxylapatite layer (5) is provided on the thermally sprayed surface layer (2).

11. An orthopedic implant in accordance with any one of claims 5 to 10, wherein the orthopedic implant is a shoulder implant, an elbow implant, a spinal column implant, a hip joint implant, a knee implant, a finger implant or any other orthopedic implant.

## Revendications

1. Couche superficielle de titane (21) pulvérisée thermiquement sur un substrat non métallique (3) d'un implant orthopédique (1), la couche superficielle pulvérisée thermiquement comportant, rapporté au titane (21), une addition sensible aux rayons X comprise entre 0,01 % en atome et 20 % en atome d'un métal indicateur biocompatible (4), le métal indicateur (4) étant un métal du groupe de métaux constitué par le tantale, l'or, le platine et le hafnium, de telle sorte qu'une masse atomique du métal indicateur (4) est plus grande que la masse atomique du titane (21), **caractérisée en ce que** la couche superficielle pulvérisée thermiquement est configurée comme matrice de titane (200) avec des zones (41) en le métal indicateur (4) incorporé.

2. Couche superficielle pulvérisée thermiquement selon la revendication 1, dans laquelle la proportion du métal indicateur (4) est comprise entre 0,5 % en atome et 5 % en atome.

3. Couche superficielle pulvérisée thermiquement selon la revendication 2, dans laquelle le métal indicateur (4) est un alliage de deux métaux du groupe de métaux constitué du tantale, de l'or, du platine et du hafnium.

4. Couche superficielle pulvérisée thermiquement selon l'une quelconque des revendications précédentes, dans laquelle la couche superficielle pulvérisée thermiquement consiste en titane (21) et en le métal indicateur (4), exception faite des impuretés.

5. Implant orthopédique comprenant un substrat non métallique (3) avec une couche superficielle (2) de titane (21) pulvérisée thermiquement, la couche superficielle (2) pulvérisée thermiquement comportant, rapporté au titane (21), une addition sensible aux rayons X comprise entre 0,01 % en atome et 20 % en atome d'un métal indicateur biocompatible (4), le métal indicateur (4) étant un métal du groupe de métaux constitué par le tantale, l'or, le platine et le hafnium, de telle sorte qu'une masse atomique du métal indicateur (4) est plus grande que la masse atomique du titane (21), **caractérisé en ce que** la couche superficielle pulvérisée thermiquement est configurée comme matrice de titane (200) avec des zones (41) en le métal indicateur (4) incorporé.

6. Implant orthopédique selon la revendication 5, dans lequel la proportion du métal indicateur (4) est comprise entre 0,5 % en atome et 5 % en atome.

7. Implant orthopédique selon la revendication 6, dans lequel le métal indicateur (4) est un alliage de deux métaux du groupe de métaux constitué du tantale, de l'or, du platine et du hafnium.

8. Implant orthopédique selon l'une quelconque des revendications 5 à 7, dans lequel la couche superficielle pulvérisée thermiquement (2) consiste en titane (21) et en le métal indicateur (4), exception faite des impuretés.

9. Implant orthopédique selon l'une quelconque des revendications 5 à 8, dans lequel le substrat (3) comprend un plastique biocompatible, en particulier du PEEK (31) et/ou un matériau à fibres de carbone (32), en particulier un matériau composite (33) de PEEK et de fibres de carbone.

10. Implant orthopédique selon l'une quelconque des revendications 5 à 9, dans lequel une couche d'hydroxyapatite (5) biocompatible est prévue sur la couche superficielle (2) pulvérisée thermiquement.

11. Implant orthopédique selon l'une quelconque des revendications 5 à 10, l'implant orthopédique étant un implant d'épaule, un implant de coude, un implant de colonne vertébrale, un implant d'articulation de la hanche, un implant de genou, un implant de doigt ou un autre implant orthopédique.
